# EUROPEAN PATENT SPECIFICATION

(11) **EP 1 367 967 B1**
(45) Date of publication and mention of the grant of the patent: **26.08.2009**
(21) Application number: 02704975.8
(22) Date of filing: 13.03.2002
(51) Int. Cl.: A61F 5/01, A61F 5/058

(54) **ORTHOPAEDIC SPLINT**
ORTHOPÄDISCHE SCHIENE
ATTELLE ORTHOPEDIQUE

(30) Priority: 13.03.2001 GB 0106111
(43) Date of publication of application: 10.12.2003
(73) Proprietor: DJO, LLC, Vista, CA 92081 (US)
(72) Inventor: Degun, Michael, Stock, Essex CM4 9LR (GB); Lennox, Iain Andrew Craig, Chelsford, Essex CM2 7TB (GB)
(74) Representative: Boyce, Conor
(86) International application number: PCT/GB2002/001154
(87) International publication number: WO 2002/071988

(56) References cited:
- EP-A- 0 770 368
- GB-A- 970 890
- US-A- 3 701 349
- US-A- 3 955 565
- US-A- 5 407 421

## Description

This invention relates to improvements in orthopaedic splints, and in particular to improvements in pneumatic splints of the type comprising rigid members associated with one or more liners which are, in use, inflated to engage the body part to which the splint is applied.

In the present context, the term "splint" is intended to mean a rigid assembly for application to a body part, most commonly a limb, so as to support and maintain parts of the limb in a constant mutual disposition, thereby facilitating repair of a fracture or other tissue damage.

An orthopaedic splint of the general type with which the present invention is concerned, intended for application to an ankle, is disclosed in US Patent No 3955565. Such a splint comprises a pair of rigid shell members which cooperate to define a shell that can be mounted about an ankle. The two shell members are fitted with touch-and-close (VELCRO-type) fasteners by which they can be held together and in position. Inflatable air-bags are disposed within the shell members such that when inflated the air-bags conform to and engage the lower limb. Each air-bag is fitted with a valve by which air may be introduced into the air-bag, but is prevented from escaping.

Developments of the device described above are disclosed in US Patent No 4628945, US Patent No 5125400 and European Patent No 0252121. US Patent No 4287920 describes a form of self-sealing valve that can be used in such devices. This valve has a mouth, the internal surfaces of which are coated with a tacky, grease-like substance. This causes the juxtaposed surfaces to adhere to each other, thereby sealing the mouth of the valve in a leak-proof manner.

Published European Patent Application EP 0770368 discloses a walker brace having air-bags that cover achilles and dorsal areas of a user's foot. This brace includes an inhalation valve that limits the pressure that can be applied to the air-bag.

There has now been devised an improvement to devices of the general type described above.

According to the invention, an orthopaedic splint comprises at least one rigid member adapted to be secured about and captivate a human or animal limb to provide support thereto and to maintain parts of the limb in a constant mutual disposition, the rigid member being associated with an inflatable air-bag, the air-bag being fitted with valve means by which air can be introduced into the air-bag and retained therewithin and by which air is released from within the air-bag if the pressure of air within the air-bag exceeds a predetermined threshold as a result of swelling of the limb after the splint has been applied thereto, characterised in that the valve means comprises a single valve unit which is used for both inflation of the air-bag and release of pressure in the event that the internal pressure rises above the pre-determined threshold.

The splint according to the invention is advantageous primarily in that it cannot be inflated to a pressure in excess of the predetermined threshold. It has been found that such excessive pressure can arise in pneumatic splints of the type known in the prior art, as a result either of over-inflation when the device is applied to the body, or as a result of oedema (swelling) that occurs subsequently. Thus, the splint can be applied and inflated until it has a tight fit with the body part. If, subsequent to fitting, the swelling in the patient's limb were to decrease, then the inflation can be "topped up", either by a medical practitioner or more commonly by the patient himself, so that tight engagement of the splint with the limb is maintained. However, if the swelling of the limb were to increase, as is commonly the case, the air-bag will automatically deflate such that the pressure remains at the predetermined threshold. The application of excessive pressure to the limb, which could have the consequence of inhibiting or cutting off the supply of blood to the limb, is thereby avoided. The splint according to the invention is therefore able to hold the injured limb tightly, even if the patient's swelling decreases, yet is safe in that it cannot be fitted too tightly. Even if further swelling occurs after fitting, the splint cannot become too tight, as it will self-deflate.

The valve means comprises a single valve unit which is used for both inflation of the air-bag and release of pressure in the event that the internal pressure rises above the pre-determined threshold.

Valves serving for both inflation and release of excessive pressure may be similar to types supplied by Halkey-Roberts Corporation of 11600 9th Street North, St Petersburg, FL 33716, USA. A particular valve suitable for inflation of the splint is the valve supplied by that company under the product number C2472000xx. The pressure relief valve may be similar to that supplied by Halkey-Roberts under the product number C24781xxxx.

Apart from the valve used for pressure relief, the device according to the invention may be generally similar to prior art devices. It may comprise one or more rigid splint members, which are most conveniently formed, eg by moulding, from suitably rigid synthetic plastics materials.

Most preferably, the splint according to the invention comprises a pair of cooperating splint members, each of generally hemi-cylindrical form, the two splint members together defining a generally tubular casing that can be fitted about a limb or part of a limb.

Where the splint according to the invention comprises more than one rigid member, the rigid members are preferably secured together by releasable fastenings. "Touch-and-close" or "hook-and-loop" (VELCRO-type) fasteners are particularly suitable for this purpose, and at least one (and more preferably several) such fasteners are therefore preferably affixed to the rigid members that are to be secured together. Such fasteners normally take the form of tapes having mating male and female components.

Preferably, the inflatable air-bag is disposed, in use, between a greater part of the rigid member and the body parts to which the splint is applied.

A single air-bag may be incorporated into the device, or there may be separate air-bags associated with some or all of several rigid splint members. There may also be more than one air-bag associated with a single splint member. Where there is more than one air-bag, each air-bag is preferably provided with valve means in accordance with the invention.

In a currently preferred embodiment, a single air-bag is provided, the air-bag having the form of a plurality of tentacles or fingers connected at one end thereof and extending longitudinally along the interior of each of two splint members that together form, in use, a generally tubular casing.

The air-bag may be of any suitably flexible and air-tight material. Plastics materials are again preferred. Most preferably, the material from which the air-bag is formed is elastic. This facilitates conformance of the inflated air-bag to the shape of the limb to which the splint is applied and the accommodation of changes in the form of the limb as it swells and then returns to normal.

The air-bag may bear directly upon the patient's skin. Preferably, however, an intermediate material, eg a soft fabric or foam material, may be interposed between the air-bag and the patient. As well as enhancing comfort for the patient, such a layer of foam material may provide for a more even distribution of pressure.

A layer of foam material is preferably also interposed between the air-bag and the internal surface of the splint member. The air-bag is thus preferably encased between two layers of foam material, one between the air-bag and the splint member and the other between the air-bag and the patient's skin.

Because the foam material is in constant contact with the patient's skin, it is preferably of a permeable material that allows the skin to "breathe". One or more openings may also be provided in the splint member to enhance flow of air to and from the patient's skin.

While the description above has focussed principally on pre-formed splints comprising one or more, preferably two or more cooperating components formed, for instance, in plastics material, it will be appreciated that the invention also encompasses rigid splints and the like formed by casting of material directly about an injured limb. In such a case, the air-bag is simply interposed between the limb and the material cast about it. Obviously, it is necessary for the valve means to be accessible to provide for inflation of the air-bag after hardening of the cast and for venting of air from the air-bag in the event that excessive pressure develops.

The splint according to the invention has been described above in terms of its application to a "limb". It will be apparent to those skilled in the art that appropriately-formed splints according to the invention can be applied to any suitable part of a limb such as the ankle, wrist, knee or elbow. The term "limb" as used herein should be interpreted to encompass all such body parts to which the splint may be applied.

The invention will now be described in greater detail, by way of illustration only, with reference to the accompanying drawings, in which
Figure 1 is an internal view of an embodiment of a wrist splint in accordance with the invention, in an opened condition prior to use;
Figure 2 is view similar to Figure 1, but showing the exterior of the splint;
Figure 3 is a view, partially in section of an inflation valve used in the splint;
Figure 4 is a sectional view of a pressure relief valve used in the splint.

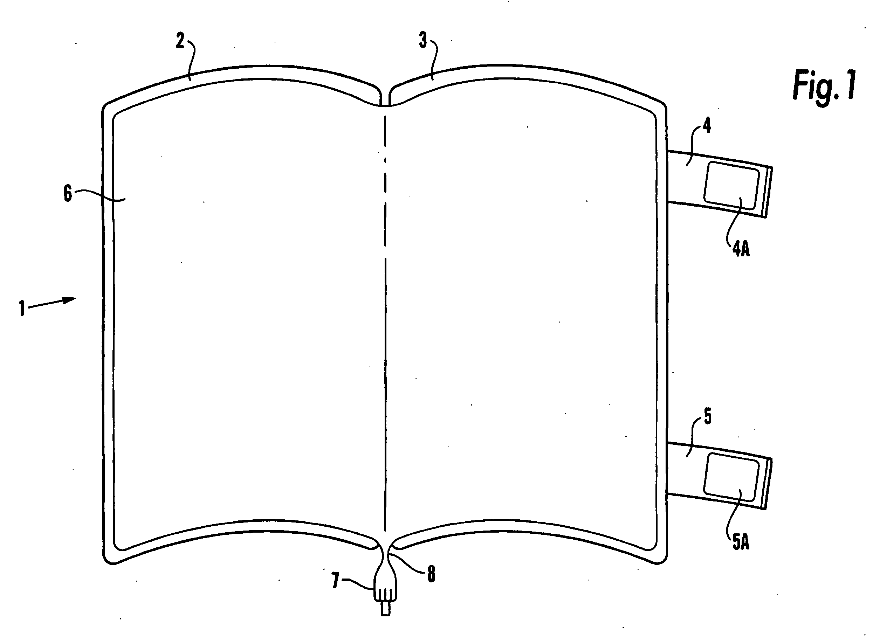

Referring first to Figure 1, an embodiment of a wrist splint in accordance with the invention is generally designated 1 and comprises a complementary pair of rigid splint members 2,3, each of which is of generally hemi-cyclindrical form and is moulded in plastics material.

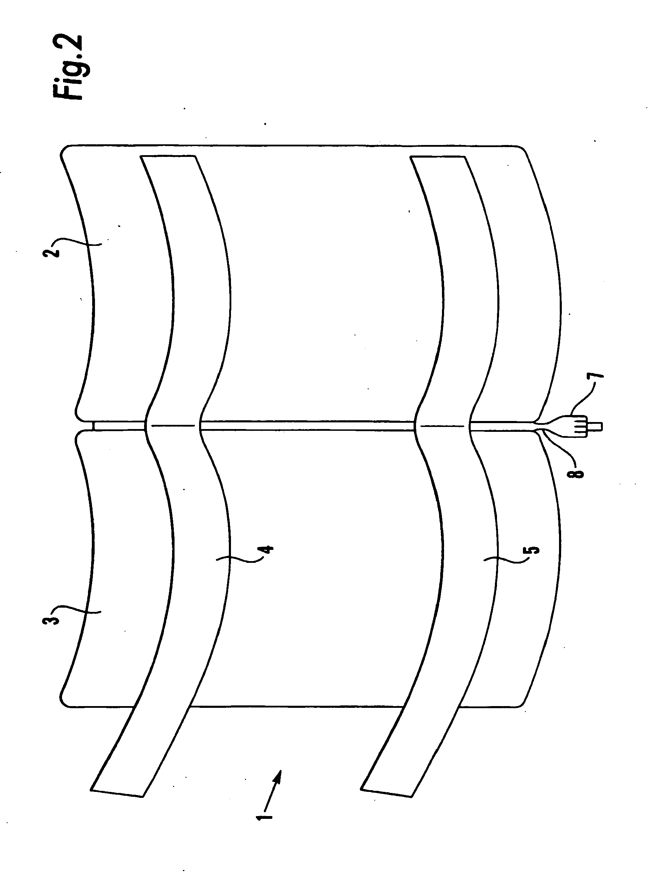

The two splint members 2,3 are hingedly connected along one major edge of each by a pair of elasticated tapes 4,5 that pass around the outside of each splint member 4,5 (see Figure 2) and are bonded to the outside surfaces of the splint members 2,3. The tapes 4,5 extend at one end beyond the edge of the right-hand (as viewed in Figure 1) edge of the splint member 3. The internally-facing side of the extending part of each tape 4,5 is provided with a patch (4a,5a respectively) of a male (hook) part of a touch-and-close fastener. The external surfaces of the tapes 4,5 have raised loop pile or plush that serves as the female (loop) part of the fastener.

The greater part of the internal surfaces of the splint members 2,3 is covered by an inflatable liner 6 which is fitted with an inflation and pressure-relief valve unit 7 which is mounted, near the hinged spine of the splint 1, on the end of short length of tubing 8 that opens into the liner 6.

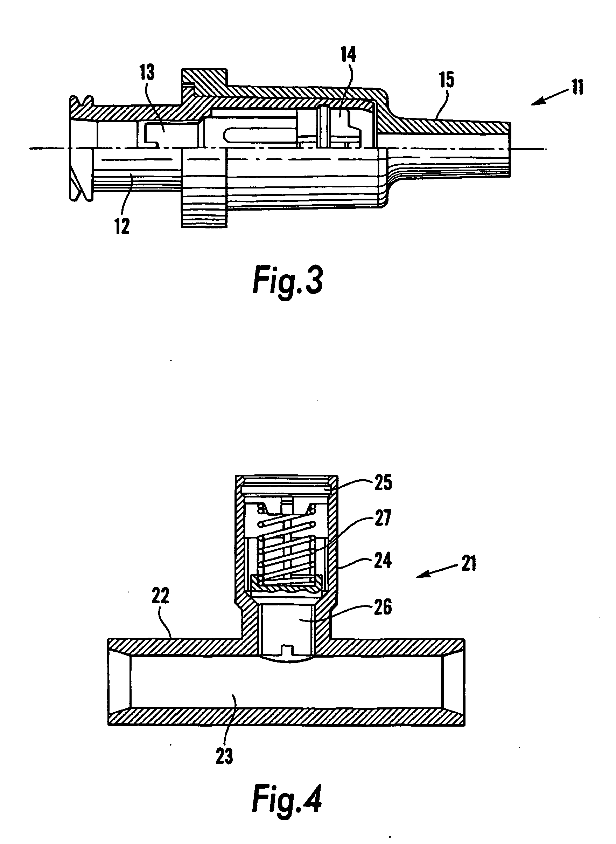

The valve unit 7 comprises two valves : an inflation valve 11 (shown in Figure 3) and a pressure relief valve 21 (see Figure 4). The inflation valve 11 is similar to that supplied by Halkey-Roberts Corporation under the product number C2472000xx, and comprises a rigid plastics body 12 containing a silicone valve stem 13 and a polypropylene plug 14. The body 12 is fitted with a male luer adapter 15.

The pressure relief valve 21 is similar to Halkey-Roberts type C24781xxxx and comprises a rigid body 22 of T-section, having a principal channel 23 from which a valve limb 24 is upstanding. The valve limb 23 houses a terminal plug 25 and a valve stem 26 between which a stainless steel compression spring 27 acts with a predetermined pressure (which is set by the characteristics of the spring 27).

The male luer adapter 15 of the inflation valve 11 is inserted into, and bonded to, one end of the principal channel 23 of the pressure relief valve 21 to form the complete valve unit 7. The other end of the principal channel 23 is connected, via the tubing 8, to the liner 6.

In use, the splint 1 is applied to an injured wrist as follows. First, the left-hand (as viewed in Figure 1) splint member 2 is positioned beneath the wrist and the other splint member 3 then folded over the first splint member 2 so that the two splint members 2,3 together encapsulate the wrist. The enclosure so formed is held together by pulling the ends of the tapes 4,5 around the abutting free edges of the two splint members 2,3 and engaging patches 4a,5a of male hooks with the plush surface of the tapes 4,5 bonded to the splint member 2.

The liner 7 is then inflated by either the fitter or the patient, either using a suitable pump attached to the inflation valve 11 or simply be blowing air through the inflation valve 11, this most conveniently being achieved by attachment to the inflation valve 11 of a tube of sufficient length to reach the patient's (or fitter's) mouth without the wrist having to be raised to a possibly uncomfortable position.

The liner 6 is inflated until the splint 1 has a close and tight fit with the wrist. If a pre-determined pressure (above which blood flow could be impaired) is exceeded, then air is automatically released through the pressure relief valve 21 until the pressure falls to that pre-determined level. Similarly, the pressure is prevented from rising in the event that further swelling of the injured wrist occurs, leading to compression of the inflated liner 6. Nonetheless, if the swelling reduces, the liner 6 can be further inflated very easily, so as to maintain the snug fit of the splint 1 with the wrist.

## Claims

1. An orthopaedic splint (1) comprises at least one rigid member (2,3) adapted to be secured about and captivate a human or animal limb to provide support thereto and to maintain parts of the limb in a constant mutual disposition, the rigid member (2,3) being associated with an inflatable air-bag (6), the air-bag (6) being fitted with valve means (11,21) by which air can be introduced into the air-bag (6) and retained therewithin and by which air is released from within the air-bag (6) if the pressure of air within the air-bag (6) exceeds a predetermined threshold as a result of swelling of the limb after the splint (1) has been applied thereto,
**characterised in that** the valve means (11,21) comprises a single valve unit (7) which is used for both inflation of the air-bag (6) and release of pressure in the event that the internal pressure rises above the pre-determined threshold.

2. A splint (1) as claimed in Claim 1, wherein the inflatable air-bag (6) is constructed to be disposed, in use, between a greater part of the rigid member (2,3) and the body parts to which the splint (1) is applied.

3. A splint (1) as claimed in any preceding claim, wherein the rigid member (2,3) is formed in rigid synthetic plastics material.

4. A splint (1) as claimed in any preceding claim, which comprises a pair of cooperating rigid members (2,3), each of generally hemi-cylindrical form, the two rigid members (2,3) together defining a generally tubular casing that can be fitted about a limb or part of a limb.

5. A splint (1) as claimed in any preceding claim, which comprises more than one rigid member (2,3), and the rigid members (2,3) are secured together by releasable fastenings (4,4a,5,5a).

6. A splint (1) as claimed in Claim 5, wherein the releasable fastenings (4,4a,5,5a) are of the hook-and-loop type with cooperating male and female components.

7. A splint (1) as claimed in any preceding claim, which comprises a single air-bag (6).

8. A splint (1) as claimed in any preceding claim, which comprises a single air-bag (6), the air-bag (6) having the form of a plurality of tentacles or fingers connected at one end thereof and extending longitudinally along the interior of each of two splint members (2,3) that together form, in use, a generally tubular casing.

9. A splint (1) as claimed in any preceding claim, wherein the air-bag (6) is formed of plastics material.

10. A splint (1) as claimed in any preceding claim, wherein the air-bag (6) is formed of elastic material.

11. A splint (1) as claimed in any preceding claim, wherein an intermediate material is interposed between the air-bag (6) and the patient's skin.

12. A splint (1) as claimed in Claim 11, wherein the intermediate material is a plastics foam material.

13. A splint (1) as claimed in Claim 12, wherein a second layer of plastics foam material is interposed between the air-bag (6) and the internal surface of the rigid member (2,3).

14. A splint (1) as claimed in any preceding claim, wherein one or more openings are provided in the rigid member (2,3) to enhance flow of air to and from the patient's skin.

15. A splint (1) as claimed in Claim 1, wherein when the rigid member (2,3) is cast about a patient's limb, the air-bag (6) is interposed between the limb and the material cast about it.

## Patentansprüche

1. Eine orthopädische Schiene (1) weist mindestens ein starres Element (2, 3) auf, das zum Befestigen um eine Gliedmaße eines Menschen oder Tiers herum und Fixieren derselben angepasst ist, um derselben Halt zu geben und Teile der Gliedmaße in einer konstanten Anordnung zueinander zu halten, wobei das starre Element (2, 3) mit einem aufblasbaren Airbag verknüpft ist, und der Airbag (6) mit einem Ventilmittel (11, 21) ausgestattet ist, durch das Luft in den Airbag (6) eingebracht und darin gehalten werden kann, und durch das Luft aus dem Innenraum des Airbags (6) abgegeben werden kann, wenn der Luftdruck innerhalb des Airbags (6) infolge von Anschwellen der Gliedmaße nach Anbringung der Schiene (1) an derselben einen vorbestimmten Grenzwert überschreitet,
**dadurch gekennzeichnet, dass** das Ventilmittel (11, 21) eine einzelne Ventileinheit (7) aufweist, die sowohl zum Aufblasen des Airbags (6) als auch zum Ablassen von Druck verwendet wird, falls der Innendruck über den vorbestimmten Grenzwert ansteigt.

2. Schiene (1) nach Anspruch 1, bei der der aufblasbare Airbag (6) konstruiert ist, um in Gebrauch zwischen einem größeren Teil des starren Elements (2, 3) und den Körperteilen angeordnet zu werden, an denen die Schiene (1) angebracht wird.

3. Schiene (1) nach einem vorhergehenden Anspruch, bei der das starre Element (2, 3) aus starrem synthetischem Kunststoffmaterial gebildet wird.

4. Schiene (1) nach einem vorhergehenden Anspruch, die ein Paar zusammenwirkender starrer Elemente (2, 3) aufweist, jeweils von allgemein halbzylindrischer Form, wobei die zwei starren Elemente (2, 3) zusammen ein allgemein röhrenförmiges Gehäuse begrenzen, das um eine Gliedmaße oder Teil einer Gliedmaße herum eingepasst werden kann.

5. Schiene (1) nach einem vorhergehenden Anspruch, die mehr als ein starres Element (2, 3) aufweist, und bei der die starren Elemente (2, 3) aneinander durch lösbare Befestigungsmittel (4, 4a, 5, 5a) befestigt werden.

6. Schiene (1) nach Anspruch 5, bei der die lösbaren Befestigungsmittel (4, 4a, 5, 5a) vom Klettverschlusstyp mit zusammenwirkenden männlichen und weiblichen Komponenten sind.

7. Schiene (1) nach einem vorhergehenden Anspruch, die einen einzigen Airbag (6) aufweist.

8. Schiene (1) nach einem vorhergehenden Anspruch, die einen einzigen Airbag (6) aufweist, wobei der Airbag (6) die Form eine Mehrzahl von Tentakeln oder Fingern aufweist, die an einem ihrer Enden verbunden sind und sich in Längsrichtung entlang des Innenraums jedes der beiden Schienenelemente (2, 3) erstrecken, welche zusammen in Gebrauch ein allgemein röhrenförmiges Gehäuse bilden.

9. Schiene (1) nach einem vorhergehenden Anspruch, bei der der Airbag (6) aus Kunststoffmaterial gebildet wird.

10. Schiene (1) nach einem vorhergehenden Anspruch, bei der der Airbag (6) aus elastischem Material gebildet wird.

11. Schiene (1) nach einem vorhergehenden Anspruch, bei der ein Zwischenmaterial zwischen dem Airbag (6) und der Haut des Patienten eingefügt wird.

12. Schiene (1) nach Anspruch 11, bei der das Zwischenmaterial ein Schaumstoffmaterial ist.

13. Schiene (1) nach Anspruch 12, bei der eine zweite Schicht aus Schaumstoffmaterial zwischen dem Airbag (6) und der Innenfläche des starren Elements (2, 3) eingefügt wird.

14. Schiene (1) nach einem vorhergehenden Anspruch, bei der eine oder mehrere Öffnungen in dem starren Element (2, 3) vorgesehen sind, um den Luftstrom zu und von der Haut des Patienten zu verbessern.

15. Schiene (1) nach Anspruch 1, bei der, wenn das starre Element (2, 3) um eine Gliedmaße eines Patienten gegossen wird, der Airbag (6) zwischen der Gliedmaße und dem um diese herum gegossenen Material eingefügt wird.

## Revendications

1. Attelle orthopédique (1) comprenant au moins un élément rigide (2, 3) adapté pour être attaché autour d'un membre humain ou animal, et le maintenir afin d'assurer son soutien et de garder les parties du membre en position mutuelle constante, l'élément rigide (2, 3) étant associé à un coussin gonflable (6), le coussin gonflable (6) étant doté d'un moyen à soupape (11, 21) par lequel on peut introduire de l'air dans le coussin gonflable (6) et l'y retenir à l'intérieur, et par lequel on peut libérer l'air de l'intérieur du coussin gonflable (6) si la pression de l'air dans le coussin gonflable (6) dépasse un seuil prédéterminé suite au gonflement du membre après que l'attelle (1) y a été appliquée,
**caractérisée par le fait que** le moyen soupape (11, 21) comprend une seule unité soupape (7) qui sert à la fois pour gonfler le coussin gonflable (6) et libérer la pression dans le cas où la pression interne dépasse le seuil prédéterminé.

2. Attelle (1) conforme à la revendication 1, où le coussin gonflable (6) est construit de manière à être disposé, à l'usage, entre une plus grande partie de l'élément rigide (2, 3) et les parties du corps auxquelles l'attelle (1) est appliquée.

3. Attelle (1) conforme à une quelconque des revendications précédentes, où l'élément rigide (2, 3) est fabriqué en matière plastique rigide synthétique.

4. Attelle (1) conforme à une quelconque des revendications précédentes, qui comporte une paire d'éléments rigides coopérants (2, 3), chacun de forme généralement semi-cylindrique, les deux éléments rigides (2, 3) délimitant ensemble une gaine généralement tubulaire pouvant être installée autour d'un membre ou d'une partie d'un membre.

5. Attelle (1) conforme à une quelconque des revendications précédentes, qui comporte plus d'un élément rigide (2, 3), et les éléments rigides (2, 3) sont attachés ensemble par des fixations libérables (4, 4a, 5, 5a).

6. Attelle (1) conforme à la revendication 5, où les fixations libérables (4, 4a, 5, 5a) sont du type à boucles et crochets avec des composants mâles et femelles coopérants.

7. Attelle (1) conforme à une quelconque des revendications précédentes, qui comprend un seul coussin d'air (6).

8. Attelle (1) conforme à une quelconque des revendications précédentes, qui comprend un seul coussin d'air (6), le coussin d'air (6) ayant la forme d'une pluralité de tentacules ou doigts connectés à une extrémité et s'étendant longitudinalement le long de l'intérieur de chacun des deux éléments de l'attelle (2, 3) qui forment ensemble, à l'usage, une gaine généralement tubulaire.

9. Attelle (1) conforme à une quelconque des revendications précédentes, où le coussin d'air (6) est fabriqué en matière plastique.

10. Attelle (1) conforme à une quelconque des revendications précédentes, où le coussin d'air (6) est fabriqué en matière élastique.

11. Attelle (1) conforme à une quelconque des revendications précédentes, où un matériau intermédiaire est interposé entre le coussin d'air (6) et la peau du patient.

12. Attelle (1) conforme à la revendication 11, où le matériau intermédiaire est un matériau en mousse plastique.

13. Attelle (1) conforme à la revendication 12, où une deuxième couche de matériau de mousse plastique est interposée entre le coussin d'air (6) et la surface interne de l'élément rigide (2, 3).

14. Attelle (1) conforme à une quelconque des revendications précédentes, où une ou plusieurs ouvertures sont prévues dans l'élément rigide (2, 3) pour augmenter la circulation d'air vers et depuis la peau du patient.

15. Attelle (1) conforme à la revendication 1, où, quand l'élément rigide (2, 3) est posé autour du membre d'un patient, le coussin d'air (6) est interposé entre le membre et le matériau posé autour de celui-ci.
